(19) Europäisches Patentamt
European Patent Office
Office européen des brevets

(11) **EP 2 864 034 B1**

(12) **FASCICULE DE BREVET EUROPEEN**

(45) Date de publication et mention de la délivrance du brevet:
**07.03.2018 Bulletin 2018/10**

(21) Numéro de dépôt: **13737338.7**

(22) Date de dépôt: **20.06.2013**

(51) Int Cl.:
*B01J 13/18* (2006.01)  *B01J 13/22* (2006.01)
*A61K 8/11* (2006.01)  *A61K 9/50* (2006.01)
*C09B 67/00* (2006.01)  *C11D 3/50* (2006.01)

(86) Numéro de dépôt international:
**PCT/FR2013/051438**

(87) Numéro de publication internationale:
**WO 2013/190240 (27.12.2013 Gazette 2013/52)**

(54) **MATÉRIAU MULTICOMPARTIMENTE POUR LA DÉLIVRANCE THERMOSTIMULÉE DE SUBSTANCES D'INTÉRÊT, PROCÉDÉ DE PRÉPARATION, APPLICATIONS**

MEHRKAMMERMATERIAL ZUR THERMISCH STIMULIERTEN FREISETZUNG VON BESTIMMTEN STOFFEN, HERSTELLUNGSVERFAHREN DAFÜR UND VERWENDUNGEN DAVON

MULTI-COMPARTMENT MATERIAL FOR THE THERMALLY STIMULATED DELIVERY OF SUBSTANCES OF INTEREST, PREPARATION METHOD THEREOF AND USES OF SAME

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.06.2012 FR 1255844**

(43) Date de publication de la demande:
**29.04.2015 Bulletin 2015/18**

(73) Titulaire: **Centre National de la Recherche Scientifique**
**75016 Paris (FR)**

(72) Inventeurs:
- **SCHMITT, Véronique**
  **F-33400 Talence (FR)**
- **NOLLET, Maxime**
  **F-33000 Bordeaux (FR)**
- **DEPARDIEU, Martin**
  **F-45000 Orleans (FR)**
- **BACKOV, Rénal**
  **F-33200 Bordeaux-Cauderan (FR)**

(74) Mandataire: **Goulard, Sophie**
**Ipsilon**
**Le Centralis**
**63, avenue du Général Leclerc**
**92340 Bourg-la-Reine (FR)**

(56) Documents cités:
**DE-A1-102008 021 005**

- **MATHIEU DESTRIBATS ET AL: "Thermostimulable Wax@SiO 2 Core-Shell Particles", LANGMUIR, vol. 26, no. 3, 2 février 2010 (2010-02-02), pages 1734-1742, XP055053549, ISSN: 0743-7463, DOI: 10.1021/la902828q**
- **DATABASE WPI Week 201066 Thomson Scientific, London, GB; AN 2010-M39814 XP002692245, & CN 101 824 307 A (UNIV BEIJING CHEM TECHNOLOGY) 8 septembre 2010 (2010-09-08)**
- **DATABASE WPI Week 199514 Thomson Scientific, London, GB; AN 1995-102158 XP002692242, & JP 7 026251 A (MITSUBISHI JUKOGYO KK) 27 janvier 1995 (1995-01-27)**
- **DATABASE WPI Week 198417 Thomson Scientific, London, GB; AN 1984-103940 XP002692243, & JP 59 046124 A (NIPPON SANSO KK) 15 mars 1984 (1984-03-15)**

# Description

La présente invention est relative à un matériau constitué d'une enveloppe de silice renfermant une phase grasse dans laquelle sont dispersées des capsules de silice renfermant une phase aqueuse, à son procédé de préparation, à son utilisation pour la délivrance thermostimulée de substances actives, ainsi qu'aux compositions renfermant un tel matériau.

Il peut être utile d'encapsuler des molécules d'intérêt telles que des médicaments, des colorants, des pigments, des réactifs, des parfums, des pesticides, etc..., pour les protéger des agressions extérieures, notamment de l'oxydation, pour les acheminer vers un lieu d'administration où elles pourront être délivrées ou bien encore pour les stocker avant une utilisation dans des conditions où elles seront libérées de leur capsule sous l'influence d'un stimulus externe. Une des premières applications de la micro encapsulation été la mise au point d'un papier copiant sans carbone commercialisé à la fin des années 60 dans lequel des microcapsules emprisonnant une encre étaient présentes sur le verso d'une feuille de papier de façon à libérer l'encre par rupture des capsules sous la pression exercée par la pointe d'un stylo lors de l'écriture. De nos jours, l'encapsulation se développe dans différents secteurs industriels tels que les industries pharmaceutique, cosmétique, alimentaire, textile et agricole. Les capsules et microcapsules deviennent de plus en plus sophistiquées, notamment dans le domaine pharmaceutique où elles permettent de faire de la délivrance contrôlée et/ou ciblée de principes actifs

Différents types et morphologies de capsules et microcapsules ont déjà été proposées tels que par exemple des capsules protéiques, des cyclodextrines, des liposomes, des vésicules lamellaires concentrées, des émulsions doubles, des colloidosomes, des microcapsules à enveloppes de silice, des nanocapsules de silice et de polymères thermosensibles tel que le poly(*N*-isopropylacrylamide (PNIPAM), des microsphères d'hydrogel thermosensible, des microsphères de PNIPAM-Polylactide, etc....De nombreuses méthodes permettant de préparer ces différents type de capsules et microcapsules ont également été développées et mises au point durant ces dernières années, telles que par exemple et de façon non exhaustive la précipitation de polymères par séparation de phase, le dépôt d'électrolyte couche par couche, la polymérisation par polycondensation interfaciale, etc...

L'inconvénient des techniques déjà connues est que la libération des molécules d'intérêt à partir des capsules et microcapsules proposées dans l'art antérieur est le plus souvent lente et progressive, c'est-à-dire prolongée dans le temps.

Il a alors été proposé de remédier à ce problème en proposant des matériaux constitués d'une enveloppe de silice renfermant un coeur de cire contenant une ou plusieurs substances d'intérêt, ces matériaux étant préparés par minéralisation d'une émulsion de Pickering, c'est-à-dire d'une émulsion de type huile-dans-eau dans laquelle la dispersion des gouttelettes d'huile dans l'eau est stabilisée par des nanoparticules colloïdales adsorbées à l'interface eau/huile (voir par exemple la demande de brevet FR-A-2 948 581). En utilisant une huile cristallisable pour préparer ces matériaux, c'est-à-dire une huile dont la température de fusion ($T_F$) est assez basse (par exemple 37°C), il est possible de préparer des matériaux dans lesquels la phase encapsulée est solide dans les conditions de stockage (par exemple à température ambiante) mais devient liquide dans les conditions d'utilisation du matériau (par exemple à une température supérieure à 37°C dans le cas d'un médicament ingérable ou injectable), provoquant ainsi la rupture de la capsule par fusion et expansion thermique de la phase encapsulée et la libération concomitante et rapide de la ou des substances actives contenues dans la phase encapsulée. Ces matériaux permettent d'encapsuler aussi bien des substances d'intérêt lipophiles (sous forme solubilisée) que des substances d'intérêt hydrophiles (sous forme dispersée). Cependant, lorsque la phase encapsulée de ces matériaux renferme plusieurs substances de nature chimique différente, celles-ci sont au contact les unes des autres ce qui ne permet pas, au sein d'un même matériau, d'encapsuler des substances pouvant présenter une incompatibilité chimique et/ou physique. De plus, ces matériaux ne permettent pas de contenir à la fois des substances hydrophiles et lipophiles toutes deux sous forme solubilisées puisque seules les substances lipophiles peuvent être présentes sous forme solubilisée dans la phase grasse encapsulée par l'enveloppe de silice alors que les substances hydrophiles y sont sous forme dispersée. Mathieu Destribats, "Thermostimulable Wax@SiO2 Core-Shell Particles", Langmuir, vol. 26, no. 3, 2 février 2010 (2010-02-02), pages 1734-1742, décrit des particules coeur-écorce de diamètre de 100 $\mu$m constituées d'une enveloppe continue comprenant le dioxyde de silicium, laditte enveloppe emprisonnant l'eicosane. Il n'existe donc pas à ce jour de système compartimenté permettant d'encapsuler sous forme solubilisée, au sein d'un même matériau, au moins deux substances d'intérêt pouvant par ailleurs éventuellement être incompatibles entre elles, lesdites substances étant lipophiles ou hydrophiles et autorisant une libération rapide et totale de ces substances d'intérêt sous l'effet d'un stimulus extérieur en conditions douces.

Le but de la présente invention est donc de proposer un matériau permettant d'encapsuler plusieurs molécules d'intérêt de façon compartimentée et qui permettent également leur libération rapide et totale sous l'influence d'un stimulus extérieur, et en particulier d'une augmentation de la température.

La présente invention a pour objet un matériau sous forme de particules solides de diamètre variant de 1 $\mu$m à 1 cm constituées d'une enveloppe continue $E_{Ext}$ comprenant au moins un oxyde de silicium, ladite enveloppe $E_{Ext}$ emprisonnant au moins une phase grasse, ledit matériau étant caractérisé en ce que ladite phase grasse est solide à la température de stockage dudit matériau et comprend de 50 à 99,9 % en masse par rapport à la masse de ladite phase grasse d'une huile cristallisable ayant une température de fusion ($T_F$) inférieure à 100°C, et en ce que ladite phase grasse renferme au moins une substance d'intérêt lipophile $S_L$ et au moins une inclusion comprenant une enveloppe continue $E_{Int}$ comprenant au moins un oxyde de silicium, ladite enveloppe $E_{Int}$ emprisonnant une phase aqueuse comprenant au moins une substance d'intérêt hydrophile $S_H$.

Selon la présente invention, on entend par « température de stockage dudit matériau », la température à laquelle le matériau conforme à la présente invention est conservé avant son utilisation. Cette température est toujours inférieure au point de fusion de l'huile cristallisable contenue dans la phase grasse. La température de stockage est 20°C. Le matériau conforme à la présente invention présente la particularité suivante : lorsque l'on soumet le matériau à une température supérieure à la température de fusion de l'huile cristallisable, on observe une expansion thermique de la phase grasse entraînant la rupture de l'enveloppe de silice $E_{Ext}$ ainsi que la rupture de la ou des enveloppes $E_{Int}$, et la libération rapide et totale de la phase grasse en fusion (c'est-à-dire à l'état liquide) comprenant la ou les substances d'intérêt $S_L$, ainsi que la libération de la phase aqueuse contenue dans les inclusions et comprenant la ou les substances $S_H$. Ce résultat est tout à fait surprenant dans la mesure où l'oxyde de silicium entrant dans la constitution de l'enveloppe est connu pour être un isolant thermique. De plus, il n'était pas du tout évident que l'expansion thermique de la phase grasse permette d'entrainer à la fois la rupture de l'enveloppe $E_{Ext}$ et celle de la ou des enveloppes $E_{Int}$.

On entend dans le cadre de cet exposé par le terme « huile cristallisable », les matières grasses et les mélanges de matières grasses, d'origine naturelle (animale ou végétale) ou synthétique, dont le point de fusion est supérieur à 15°C, de préférence dont le point de fusion varie de 20 à 100°C environ, et en particulier de 20 à 50°C environ. Tous les points de fusion mentionnés dans la description de la présente demande font référence à des points de fusion déterminés par calorimétrie différentielle à balayage à pression atmosphérique ou « *Differential Scanning Calorimetry* » (DSC) en anglais.

L'huile cristallisable forme une partie majoritaire de la phase grasse et peut même, outre la ou les substances d'intérêt $S_L$ et les inclusions, être l'unique constituant de celle-ci. L'huile cristallisable représente de préférence de 75 à 99,9 % en masse environ par rapport à la masse de la phase grasse.

Le choix de l'huile cristallisable dépend naturellement de l'application envisagée pour le matériau et donc de la température à laquelle on souhaite observer l'expansion thermique de la phase grasse et par voie de conséquence la rupture des enveloppes de silice $E_{Ext}$ et $E_{Int}$. Parmi les huiles cristallisables utilisables selon l'invention, on peut notamment citer les paraffines telles que les paraffines ayant un point de fusion entre 42 et 44°C ou entre 46 et 48°C [RN-8002-74-2] vendues par la société Merck, ; les triglycérides ; les acides gras ; les colophanes ; les cires (alcanes longs) tels que l'eicosane et l'octadécane ; les huiles végétales hydrogénées ainsi que leurs mélanges ; et les bitumes synthétiques. Ces huiles peuvent être utilisées seules ou en mélanges.

Le matériau conforme à la présente invention se présente de préférence sous la forme d'une poudre de particules sphériques ou sensiblement sphériques.

Le diamètre des particules varie de préférence de 5 $\mu$m à 500 $\mu$m environ, et encore plus préférentiellement de 10 à 200 $\mu$m.

L'enveloppe de silice $E_{Ext}$ doit avoir une épaisseur suffisante pour avoir une résistance mécanique permettant l'encapsulation de la phase grasse. Cependant, les enveloppes de silice $E_{Ext}$ et $E_{Int}$ doivent également présenter une épaisseur leur permettant de se rompre lors d'une élévation de la température à une température supérieure à la température de fusion de la phase grasse. L'épaisseur de l'enveloppe de silice $E_{Ext}$ varie généralement de 0,1 à 2 $\mu$m environ, et préférentiellement de 0,2 à 2 $\mu$m environ.

L'épaisseur de la ou des enveloppes de silice $E_{Int}$ varie généralement de 0,1 à 1 $\mu$m environ, et préférentiellement de 0,2 à 0,8 $\mu$m environ.

En plus de l'oxyde de silicium, les enveloppes $E_{Ext}$ et/ou $E_{Int}$ peuvent en outre comprendre un ou plusieurs oxydes métalliques de formule $MeO_2$ dans laquelle Me est un métal choisi parmi Zr, Ti, Th, Nb, Ta, V, W et Al. Dans ce cas, les enveloppes $E_{Ext}$ et $E_{Int}$ sont constituées d'une matrice mixte de type $SiO_2$-$MeO_2$ dans laquelle la teneur en $MeO_2$ reste minoritaire par rapport à la teneur en oxyde de silicium, préférentiellement la teneur en $MeO_2$ représente de 1% à 40% massique, plus particulièrement de 5% à 30% massique par rapport à la masse totale de l'enveloppe.

Parmi les substances d'intérêt pouvant être incorporées dans la phase grasse et dans la phase aqueuse de la ou des inclusions du matériau conforme à la présente invention, on peut notamment citer les médicaments (principes actifs), les principes actifs utilisables en cosmétique, les réactifs chimiques, les colorants, les pigments, les encres, etc ... Bien entendu, ces substances sont incorporées dans la phase grasse lorsqu'elles sont lipophiles et dans la phase aqueuse de la ou des inclusions lorsqu'elles sont hydrophiles, l'homme du métier sachant discriminer le caractère lipophile ou hydrophile d'une substance donnée en fonction de sa valeur HLB (« *Hydrophilic-Lipophilic Balance* » : balance hydrophile/hydrophobe).

A titre d'exemples de médicaments, on peut mentionner les bactéricides tels que les antiseptiques et les antibiotiques, les antis inflammatoires, les analgésiques, les laxatifs locaux, les hormones, les protéines, etc...

A titre d'exemples de principes actifs cosmétiques, on peut notamment citer les vitamines, les filtres solaires, les antioxydants tels que les antiradicalaires comme la superoxyde dismutase, les parfums, les agents absorbeurs d'odeur, les agents déodorants, les agents anti transpirants, les colorants, les pigments, les émollients, les agents hydratants, etc...

A titre d'exemples de réactifs chimiques, on peut notamment citer, les réactifs colorés, les indicateurs colorés tels que les indicateurs de pH, les catalyseurs, les amorceurs de polymérisation, les monomères, les complexants, etc...

La ou les substances d'intérêt $S_L$ représentent généralement de 0,001 à 50 % en masse environ, et préférentiellement de 0,01 à 25% en masse environ de la masse totale de la phase grasse.

La ou les substances d'intérêt $S_H$ représentent généralement de 0,001 à 50 % en masse environ, et préférentiellement de 0,01 à 25% en masse environ de la masse totale de la phase aqueuse contenue dans la ou les inclusions présentes au sein de la phase grasse.

La phase grasse et/ou la phase aqueuse de la ou des inclusions peuvent en outre renfermer un ou plusieurs additifs classiquement utilisés dans les émulsions et parmi lesquels on peut notamment mentionner à titre d'exemple les protecteurs ou de conservation de la substance d'intérêt, tels que les antioxydants, les agents anti-UV, etc....

L'invention a également pour objet un procédé de préparation du matériau tel que défini ci-dessus. Ce procédé est caractérisé en ce qu'il comporte les étapes suivantes consistant :

1) dans une première étape, à porter une phase grasse comprenant de 50 à 99,9 % en masse par rapport à la masse de ladite phase grasse d'une huile cristallisable (HC) solide ayant une température de fusion $T_F$ inférieure à 100°C à une température $T_{HC}$ telle que $T_{HC}$ est supérieure à $T_F$, pour obtenir une huile cristallisable à l'état liquide ;

2) dans une deuxième étape, à incorporer à la phase grasse à l'état liquide au moins une substance d'intérêt lipophile ($S_L$) et des particules solides colloïdales P1 ;

3) dans une troisième étape, à mettre en contact ladite phase grasse à l'état liquide avec une première phase aqueuse (PA1) préalablement portée à une température $T_{PA1}$ telle que $T_{PA1}$ est supérieure à $T_F$, ladite phase aqueuse renfermant en outre au moins une substance d'intérêt hydrophile ($S_H$) ;

4) dans une quatrième étape, à soumettre le mélange liquide résultant de la troisième étape à une agitation mécanique pour obtenir une émulsion eau-dans-huile (E/H) formée de gouttelettes de phase aqueuse dispersées dans la phase grasse continue à l'état liquide et dans laquelle les particules solides colloïdales P1 sont présentes à l'interface formée entre la phase grasse continue et les gouttelettes de phase aqueuse PA1 dispersées ;

5) dans une cinquième étape, à ajouter l'émulsion E/H obtenue ci-dessus à l'étape précédente, à une seconde phase aqueuse (PA2), préalablement portée à une température $T_{PA2}$ telle que $T_{PA2}$ est supérieure à $T_F$ et renfermant en outre des particules solides colloïdales P2, ladite émulsion E/H représentant au maximum 20% en masse par rapport à la masse de la seconde phase aqueuse,

6) dans une sixième étape, à soumettre le mélange liquide résultant de la cinquième étape à une agitation mécanique pour obtenir une émulsion double eau-dans-huile-dans-eau (E/H/E) formée d'une phase aqueuse continue (PA2) renfermant des gouttelettes de phase grasse à l'état liquide, chacune desdites gouttelettes de phase grasse à l'état liquide renfermant au moins une gouttelette de phase aqueuse PA1 ; émulsion double dans laquelle les particules solides colloïdales P2 sont présentes à l'interface formée entre la phase aqueuse continue PA2 et les gouttelettes de phase grasse à l'état liquide dispersées ;

7) dans une septième étape, à porter ladite émulsion double E/H/E à une température $T_{E/H/E}$ telle que $T_{E/H/E}$ est inférieure à $T_F$ pour provoquer la solidification de la phase grasse et obtenir une émulsion double E/H/E formée de globules de phase grasse à l'état solide, chacun desdits globules renfermant au moins une gouttelette de phase aqueuse PA1, lesdits globules étant dispersés dans la phase aqueuse PA2 ;

8) dans une huitième étape, à ajouter, dans la phase aqueuse PA2 de l'émulsion double, et sous agitation mécanique, au moins un précurseur d'oxyde de silicium et une quantité suffisante d'au moins un acide pour amener les phases aqueuses PA1 et PA2 à un pH inférieur ou égal à 4 ;

9) dans une neuvième étape, à laisser l'émulsion double E/H/E au repos, pour permettre l'hydrolyse du précurseur d'oxyde de silicium et sa condensation sous la forme d'une enveloppe d'oxyde de silicium autour desdites gouttelettes de phase aqueuse PA (minéralisation de l'émulsion) et ainsi obtenir le matériau attendu ;

10) dans une dixième étape, à séparer ledit matériau de la phase aqueuse PA2.

Les particules solides colloïdales P1 ajoutées dans la phase grasse lors de la deuxième étape et les particules P2 présentes dans la phase aqueuse PA2 lors de la cinquième étape peuvent être minérales ou organiques. De préférence, il s'agit de particules minérales choisies dans le groupe des oxydes, hydroxydes et sulfates de métaux. Parmi de tels oxydes, on peut tout particulièrement citer les oxydes de silicium, de titane, de zirconium et, de fer, ainsi que leurs sels tels que les silicates (par exemple les argiles). Enfin, on peut citer les particules colloïdales de carbone. Parmi les particules solides colloïdales organiques, on peut citer notamment les particules polymériques, par exemple des particules de latex.

Afin d'être colloïdales, les particules solides P1 et P2 présentent généralement une taille inférieure à quelques micromètres. Ainsi les particules présentent généralement une taille moyenne comprise entre 5 et 5000 nm, et de préférence entre 5 et 500 nm.

Selon une forme de réalisation particulièrement préférée de l'invention, les particules solides colloïdales P1 et P2 sont de nature chimique identique et sont choisies parmi les nanoparticules d'oxyde de silicium. A titre d'exemple, on peut notamment citer les produits vendus sous la dénomination commerciale Aerosil ® par la société Evonik Degussa. Dans ce cas, les particules P1 et P2 peuvent être de diamètre identique ou différent. On peut par exemple utiliser, à titre de particules P1, des particules d'oxyde de silicium de diamètre moyen d'environ 12 nm, et à titre de particules P2, des particules d'oxyde de silicium de diamètre moyen d'environ 16 nm.

La quantité de particules solides colloïdales P1 varie généralement de 0,01 % à 10%, en particulier de 0,1% à 7% en masse par rapport à la masse totale de la phase aqueuse PA1.

Avantageusement, la quantité de particules solides colloïdales P1 présentes dans la phase grasse varie en fonction de la taille moyenne en volume des gouttelettes de phase aqueuse PA1 souhaitées dans l'émulsion et dont le diamètre moyen varie de 0,1 $\mu$m à 100 $\mu$m, de préférence de 1 à 50 $\mu$m, et encore plus préférentiellement de 5 à 20 $\mu$m environ.

La quantité de particules solides colloïdales P2 varie généralement de 0,01 % à 5%, en particulier de 0,5 % à 2% en masse par rapport à la masse totale de l'émulsion E/H.

Avantageusement, la quantité de particules solides colloïdales P2 présentes dans la phase aqueuse PA2 varie en fonction de la taille moyenne en volume des gouttelettes de phase grasse dispersées souhaitées dans l'émulsion et dont le diamètre moyen varie de 15 à 100 $\mu$m, de préférence de 30 à 80 $\mu$m, et encore plus préférentiellement de 40 à 70 $\mu$m environ.

Par ailleurs, les particules solides colloïdales (P1 et P2) présentent généralement une surface hydrophile et chargée, ce qui ne favorise pas leur adsorption à la surface des gouttelettes de la phase aqueuse PA1 dispersée ni à la surface des gouttelettes de phase grasse dispersée dans la phase aqueuse PA2.

Ainsi, et selon une forme de réalisation préférée de l'invention, les particules solides colloïdales P1 et P2 sont fonctionnalisées en surface pour favoriser leur adsorption à l'interface formée entre la phase grasse continue et la phase aqueuse PA1 lors de l'étape 4) ou entre la phase aqueuse PA2 et les gouttelettes de phase grasse dispersées lors de l'étape 6).

Les particules solides colloïdales P1 et P2 peuvent ainsi être fonctionnalisées par des composés liés à leur surface par des liaisons covalentes. Cela peut être réalisé par traitement préalable des particules, en particulier par greffage chimique d'un composé comportant des groupements hydrophobes tel qu'un trialkoxysilane de formule R-Si-(OR')$_3$, dans laquelle R est un alkyle linéaire ou ramifié en C$_1$ à C$_{12}$, en particulier de C$_2$ à C$_{10}$, tout particulièrement n-octyle, portant éventuellement un groupe amino et R', identique ou différent de R, est un groupe alkyle linéaire ou ramifié en C$_1$ à C$_{12}$, en particulier de C$_1$ à C$_6$, et tout particulièrement éthyle.

Les particules solides colloïdales P1 et P2 peuvent également être fonctionnalisées par adsorption de molécules de tensioactif à leur surface qui permettent de leur conférer une certaine hydrophobie, l'extrémité hydrophile du tensioactif étant adsorbée sur la surface des particules. Les tensioactifs utilisables pour fonctionnaliser les particules P1 et P2 sont de préférence des tensioactifs cationiques ou anioniques.

Parmi ces tensioactifs, on préfère en particulier les alkylsulfates de sodium tels qu'en particulier le dodécylsulfate de sodium (SDS) et les bromures d'alkyltriméthylammonium.

Le tensioactif est de préférence choisi parmi les tensioactifs de charge opposée à celle de la surface des particules solides colloïdales P1 et P2. Ce choix permet de favoriser l'adsorption du tensioactif à la surface des particules.

A titre d'exemple de particules fonctionnalisées par un tensioactif, on peut notamment citer les nanoparticules de silice dont la surface est fonctionnalisée par un ammonium quaternaire telles que celles vendues sous la dénomination Aerosil® A380 par la société Evonik Degussa, de diamètre 7 nm, et dont la surface est fonctionnalisée par le bromure de cétyl-trimethylammonium (CTAB), les nanoparticules de silice de 12 nm de diamètre, vendues sous la dénomination Aerosil® AR 816 et les nanoparticules de silice de 16 nm de diamètre, vendues sous la dénomination Aerosil® AR 972 par la société Evonik Degussa.

La fonctionnalisation des particules solides colloïdales P1 et P2 par un tensioactif peut également être réalisée *in situ,* c'est-à-dire lors de leur introduction dans la phase grasse et respectivement dans la phase aqueuse PA2 de l'émulsion. Dans ce cas, la phase grasse et la phase aqueuse PA2 de l'émulsion renferment en outre ledit tensioactif en une quantité préférentiellement inférieure à la concentration micellaire critique (CMC), celui-ci venant alors s'adsorber à la surface des particules lorsque celles-ci sont dans la phase aqueuse de l'émulsion. De préférence, la quantité de tensioactif varie de 1/200$^{ème}$ à 1/3 de la CMC.

Les phases aqueuses PA1 et PA2 comprennent principalement de l'eau et éventuellement un alcool, tel que le méthanol, l'éthanol, l'isopropanol, ou le butanol, de préférence l'éthanol.

Les agitations mécaniques réalisées lors des quatrième et sixième étapes peuvent notamment être réalisée dans un dispositif destiné à émulsionner tel que par exemple dans des dispositifs vendus sous les dénominations commerciales Ultra-Turrax® ou Rayneri®.

La distribution de taille des gouttelettes de la phase aqueuse PA1 dans l'émulsion E/H est généralement étroite (U<40%).

Lors de la neuvième étape (minéralisation de l'émulsion), l'ajout d'au moins un précurseur d'oxyde de silicium à pH acide provoque la condensation dudit précurseur à l'interface formée entre les globules de phase grasse à l'état solide et la phase aqueuse PA2, ainsi qu'à l'interface entre les gouttelettes de phase aqueuse PA1 et la phase grasse. Il y a donc formation simultanée d'une enveloppe de silice autour de chacune des gouttelettes de phase aqueuse PA1 contenue dans chacune des gouttelettes de phase grasse, ainsi que d'une enveloppe de silice autour de chacune des gouttelettes de phase grasse.

Les précurseurs d'oxyde de silicium peuvent être choisis parmi les alcoxydes de silicium et en particulier parmi le tetraméthoxyorthosilane (TMOS), le tetraéthoxyorthosilane (TEOS), le diméthyldiéthoxysilane (DMDES), le (3-mercaptopropyl)triméthoxysilane, le (3-aminopropyl)triéthoxysilane, le N-(3-triméthoxysilylpropyl)pyrrole, le 3-(2,4-dinitrophénylamino)-propyltriéthoxysilane, le N-(2-aminoéthyl)-3-aminopropyltriméthoxysilane, le phényltriéthoxysilane, le méthyltriéthoxysilane et leurs mélanges. Parmi ces précurseurs, le TEOS est particulièrement préféré. Ces précurseurs peuvent être substitués, de manière totale ou partielle, par des sols de silicate.

L'épaisseur de l'enveloppe formée autour de chaque gouttelette de phase aqueuse PA1 ainsi qu'autour de chaque gouttelette de phase grasse dépend de la quantité de précurseurs d'oxyde de silicium utilisée lors de la huitième étape et du diamètre des globules de la phase grasse dispersée ainsi que du diamètre des gouttelettes de phase aqueuse PA1 dispersées dans la phase grasse. Cette quantité est exprimée par rapport à la surface totale en $m^2$ des globules de la phase dispersée de l'émulsion et des gouttelettes de phase aqueuse PA1 dispersées.

Selon une forme de réalisation préférée de l'invention, la quantité de précurseur d'oxyde de silicium varie de 0,005 à 4 $M/m^2$, et encore plus préférentiellement de 0,01 à 2,2 M par $m^2$ de surface totale des globules de la phase dispersée et des gouttelettes de phase aqueuse PA1 de l'émulsion.

Pour atteindre les plus grandes épaisseurs d'enveloppes, les étapes 8 à 10 peuvent être réalisées plusieurs fois jusqu'à obtenir l'épaisseur désirée.

Lorsque les enveloppes de silice du matériau conforme à l'invention comprennent, outre l'oxyde de silicium, un oxyde métallique, on ajoute alors également à aux phases aqueuses PA1 et PA2 de l'émulsion au moins un précurseur d'un oxyde métallique de formule $MeO_2$, ledit précurseur étant choisi parmi les alcoxydes, les chlorures ou les nitrates des métaux choisis parmi Zr, Ti, Th, Nb, Ta, V, W et Al.

Lorsqu'ils sont utilisés, la quantité de ces précurseurs d'oxyde métallique de formule $MeO_2$ varie de 0,001 M à 1 M, et préférentiellement de 0,01 à 0,6 M par $m^2$ de surface totale des globules de la phase dispersée et des gouttelettes de phase aqueuse PA1 de l'émulsion.

Le pH des phases aqueuses PA1 et PA2 lors de la huitième étape varie de préférence de 0,01 à 4, et encore plus préférentiellement de 0,1 à 2,1.

L'acide utilisé pour ajuster le pH des phases aqueuses PA1 et PA2 peut être choisi parmi les acides minéraux et organiques parmi lesquels on peut en particulier citer l'acide chlorhydrique, l'acide acétique, l'acide nitrique et l'acide sulfurique.

Lors de la dixième étape, le matériau conforme à l'invention peut être séparé de la phase aqueuse et récupéré par toute technique classique de séparation connue de l'homme du métier, telle que la filtration, la centrifugation et l'utilisation de tamis. Il est ensuite de préférence lavé, par exemple à l'eau, puis séché par exemple par lyophilisation pour donner une poudre.

Le matériau ainsi obtenu est stable au stockage pendant plusieurs mois à condition que la température de stockage soit inférieure à la température $T_F$ de la phase grasse emprisonnée dans l'enveloppe de silice.

Le matériau conforme à l'invention peut être utilisé sous forme de poudre ou de dispersion dans un solvant pour délivrer la ou les substances d'intérêt lipophile(s) présente(s) dans la phase grasse solide emprisonnée dans l'enveloppe à base de silice, ainsi que la ou les substances d'intérêt hydrophile(s) présente(s) dans les inclusions de phase aqueuse PA1, ladite phase aqueuse PA1 étant elle-même emprisonnée et séparée de la phase grasse par une enveloppe de silice.

L'invention a donc également pour objet l'utilisation d'un matériau conforme à l'invention et tel que décrit précédemment pour la délivrance thermostimulée et simultanée d'au moins une substance d'intérêt lipophile et d'au moins une substance d'intérêt hydrophile.

La délivrance des substances d'intérêt lipophile et hydrophile est obtenue par expansion thermique de la phase grasse induisant la rupture de l'enveloppe entourant la phase grasse, ainsi que la rupture de chacune des enveloppes de silice entourant chacune des gouttelettes de phase aqueuse PA1 dispersées dans la phase grasse, sous l'effet d'une élévation de la température du matériau à une température de délivrance $T_D$ telle que $T_D > T_F$.

A titre d'exemple, et lorsque les substances d'intérêt sont des principes actifs lipophile et hydrophile à action médicamenteuse ou des ingrédients actifs de compléments alimentaires tels que par exemples des vitamines lipophile et hydrophile, l'huile cristallisable présente dans la phase grasse est de préférence choisie parmi les huiles cristallisables ayant un point de fusion inférieur à 37C°. Ainsi, lorsque ledit matériau est incorporé dans une composition pharmaceutique et que cette composition est administrée à un patient, par exemple par voie orale, la composition ingérée va se trouver à la température du corps, en général 37°C ou plus, ce qui va entraîner la fusion de la phase grasse et son expansion volumique et ainsi la rupture des enveloppes de silice et la délivrance des principes actifs ou des ingrédients actifs.

Selon un autre exemple, les substances d'intérêt lipophile et hydrophile sont des principes actifs cosmétique et le matériau fait partie des composants d'une composition cosmétique à application topique, telle qu'une poudre, une crème ou un gel. L'échauffement de la phase grasse du matériau à une température supérieure à $T_F$ peut dans ce cas être provoqué par un frottement local lors de l'étalement de la composition cosmétique, ce qui induit un échauffement local entraînant la rupture des enveloppes et la libération locale des substances d'intérêt. Si la composition cosmétique se présente sous la forme d'une poudre, son application par étalement peut s'accompagner d'un changement de texture (transformation de la poudre en une composition ayant un toucher gras dû à la rupture de l'enveloppe).

A titre d'autres exemples d'utilisation du matériau conforme à l'invention, on peut notamment citer :

- l'utilisation pour la délivrance simultanée d'amorceurs lipophile et hydrophile d'une réaction de polymérisation induite par élévation de la température ; dans ce cas, le matériau conforme à l'invention peut par exemple être utilisé pour la fabrication de mousses solides dans le domaine des matériaux isolants.

L'invention a également pour objet l'utilisation du matériau tel que décrit ci-dessus, à titre d'ingrédient, pour la préparation de produits pharmaceutiques, cosmétiques ou alimentaires, ainsi que les produits pharmaceutiques, cosmétiques ou alimentaires, renfermant, à titre d'ingrédient, au moins un matériau conforme à l'invention.

Ces compositions peuvent renfermer les supports pharmaceutiques, cosmétiques ou alimentaires classiques et bien connus de l'homme du métier, ainsi qu'un ou plusieurs tensioactifs destinés à favoriser la libération de la phase grasse liquide et de la phase aqueuse encapsulée lors de la rupture des différentes enveloppes de silice.

La présente invention est illustrée par les exemples de réalisation suivants, auxquels elle n'est cependant pas limitée.

**EXEMPLES**

Les matières premières utilisées dans les exemples qui suivent sont listées ci-après :

- Eicosane pur à 99% (point de fusion = 37C°) vendu par la société Sigma Aldrich ;

- Tetraéthoxyorthosilane pur à plus de 99 % (TEOS), Rhodamine B : société Sigma-Aldrich ;

- Bromure de cétyl-triméthylammonium (CTAB) : société ChemPur ;

- Nanoparticules de silice de 12 nm de diamètre, vendues sous la dénomination Aerosil® AR 816 et nanoparticules de silice de 16 nm de diamètre, vendues sous la dénomination Aerosil® AR 972 : société Evonik Degussa.

Ces matières premières ont été utilisées telles que reçues des fabricants, sans purification supplémentaire.

La concentration micellaire critique (CMC) du CTAB dans l'eau pure à température ambiante est de 0,92 mM.

Les matériaux obtenus ont été caractérisés à l'aide d'un microscope optique inversé vendu sous la dénomination commerciale Axiovert® X100 par la société Zeiss et équipé d'une platine chauffante de la société Mettler permettant de contrôler la température ainsi que les vitesses de chauffage et de refroidissement.

La distribution de taille des émulsions a été étudiée à l'aide d'un granulomètre vendu sous la dénomination commerciale Mastersizer Hydro MS2000 par la société Malvern Instrument. Les mesures granulométriques ont été faites à 25°C dans l'eau pure. L'intensité de la diffusion en fonction de l'angle qui a été collectée a été transformée en utilisant la théorie de Mie-Lorenz. La distribution de la taille des particules a été exprimée par leur diamètre moyen pondéré (D) et leur polydispersité (P) en appliquant les équations (1) et (2) suivantes :

$$D = \frac{\sum_i N_i D_i^3}{\sum_i N_i D_i^2} \qquad (1)$$

et

$$P = \frac{1}{\overline{D}} \frac{\sum_i N_i D_i^3 |\overline{D} - D_i|}{\sum_i N_i D_i^3} \qquad (2)$$

dans lesquelles :

- $D_i$ est le diamètre des particules,
- $N_i$ est le nombre total de gouttelettes de diamètre $D_i$,
- $\overline{D}$ est le diamètre médian, c'est-à-dire l'ouverture théorique du tamis telle que 50 % des particules, en masse, ont un diamètre supérieur et 50 % un diamètre inférieur.

Ces formules sont appliquées dans les granulomètres de la société Malvern Instrument.

Les matériaux ont été observés à l'aide d'un microscope confocal vendu par la société Leica sous la dénomination True Confocal Scanner Leica TCS SP2.

**Exemple 1 : Préparation, caractérisations et étude d'un matériau conforme à l'invention**

Dans cet exemple on illustre la préparation, la caractérisation et l'étude d'un matériau conforme à l'invention, constitué d'une enveloppe de silice renfermant une huile cristallisable comprenant des gouttelettes de phase aqueuse dispersée, chacune desdites gouttelettes étant elle-même entourée d'une enveloppe de silice.

Il est à noter que dans cet exemple, les phases huileuse et aqueuse ne renferment pas de substances d'intérêt, cet exemple étant donné pour démontrer la faisabilité structurelle du matériau compartimenté selon le procédé conforme à l'invention.

Il est facile d'extrapoler le procédé ci-après à des phases huileuse et aqueuse renfermant respectivement au moins une substance d'intérêt lipophile et au moins une substance d'intérêt hydrophile. La Rhodamine B utilisée ci-après dans la phase aqueuse afin de démontrer la formation des gouttelettes de phase aqueuse entourées d'une enveloppe de silice au sein de la phase huileuse peut d'ailleurs être considérée comme une substance d'intérêt hydrophile.

1) Préparation du matériau

i) : Fonctionnalisation des particules de silice

5 g de nanoparticules de silice Aerosil ® AR 816 ont été dispersés dans 100 ml d'eau distillée, à l'aide d'une cuve à ultra sons. On a ensuite ajouté à cette dispersion, 9 mg de CTAB/g de particules, cette quantité représentant environ 1,35 fois celle de la concentration micellaire critique (CMC = 0,92 mM). La surface des nanoparticules de silice étant chargée négativement, le CTAB (tensioactif cationique) vient s'adsorber à la surface des particules de silice et permet ainsi de leur conférer un caractère amphiphile. On a obtenu une phase aqueuse PA1 renfermant une dispersion de nanoparticules de silice fonctionnalisées en surface.

La quantité de CTAB a été adaptée à la masse des particules de silice afin d'obtenir une couverture spécifique de 12 nm$^2$/molécule de CTAB à l'interface silice/eau en considérant que tout le CTAB utilisé est adsorbé à la surface des particules de silice.

ii) Préparation de l'émulsion E/H

On a introduit 20 % en masse d'une phase aqueuse PA1 contenant 0,02 % en masse de Rhodamine B et 0,1 M de NaCl préalablement chauffée à une température de 45°C dans la phase grasse (Eicosane) également préalablement chauffée à 45°C et renfermant une dispersion de particules de silice AR 972. La quantité de particules de silice AR 972 à disperser dans la phase grasse est calculée en fonction de la quantité de phase aqueuse, à raison de 50 mg de particules de silice par gramme de phase aqueuse.

L'émulsification de la phase grasse et de la phase aqueuse a été réalisée à l'aide d'un agitateur vendu sous la dénomination Ultra-Turrax ® T25 par la société Janke & Kunkel, équipé d'un outil de dispersion S25 KV-25F, en terminant par une agitation à 12000 tours/min. pendant 1 minute. L'émulsion résultante a été maintenue à 45°C dans un bain thermostaté sans agitation afin de laisser le phénomène de coalescence limité se produire (adsorption des particules de silice à la surface des gouttelettes de phase aqueuse dispersée, ce qui permet d'améliorer l'homogénéité de la distribution de la taille des gouttelettes d'eau dispersée dans l'huile).

iii) Préparation de l'émulsion double E/H/E

20 % en masse de l'émulsion E/H obtenue ci-dessus à l'étape précédente ont ensuite été ajoutés à une phase aqueuse PA2 contenant 0,1 M de NaCl et les particules de silice AR 816 fonctionnalisées telles que préparées ci-dessus. La quantité de particules fonctionnalisées dépend de la masse totale de l'émulsion E/H utilisée. Dans cet exemple, la phase aqueuse PA2 renfermait 8 mg de particules fonctionnalisées par gramme d'émulsion E/H introduite dans la phase aqueuse PA2.

L'émulsification de l'émulsion E/H dans la phase aqueuse PA2 a été réalisée à l'aide du même agitateur que ci-dessus à l'étape ii) en finissant par une agitation à 8000 tours/min. pendant 2 minutes. L'émulsion double E/H/E ainsi obtenue a ensuite été conservée dans un bain thermostaté à 45°C sans agitation pendant quelques minutes pour laisser le phénomène de coalescence limité se produire (adsorption des particules de silice à la surface des gouttelettes de phase grasse dispersée).

L'émulsion a ensuite été sortie du bain thermostaté, puis laissée revenir à température ambiante afin de provoquer la solidification de la phase grasse.

iv) Minéralisation de l'émulsion : formation des enveloppes de silice

L'émulsion double E/H/E obtenue ci-dessus à l'étape précédente a été diluée jusqu'à 7% en masse par addition d'une solution aqueuse contenant 0,5% en masse de CTAB, 46% en masse d'acide chlorhydrique (HCl) à 37 % en volume, et 0,1 M de NaCl, en conditions froides, c'est-à-dire à une température de 4°C.

Le pH du mélange ainsi formé était proche de 0, ce qui permet ensuite de réaliser l'hydrolyse et la condensation du TEOS aux interfaces huile/eau.

La minéralisation de l'émulsion a ainsi été réalisée en introduisant 10 mL de l'émulsion double acidifiée dans des tubes à essai puis en y ajoutant 0,3 g de TEOS par tube.

Les tubes ont été soumis à une agitation continue sur une roue tournante à raison de 9 tours par minute dans une chambre thermostatée à 25°C. Au bout d'une heure, le matériau attendu a été récupéré par centrifugation et lavé plusieurs fois avec de l'eau contenant 0,1 M de NaCl.

Le matériau obtenu a été conservé dans l'eau pure pendant plusieurs mois. Aucune altération des capsules n'a été observée pendant cette période.

2) Résultats et caractérisations

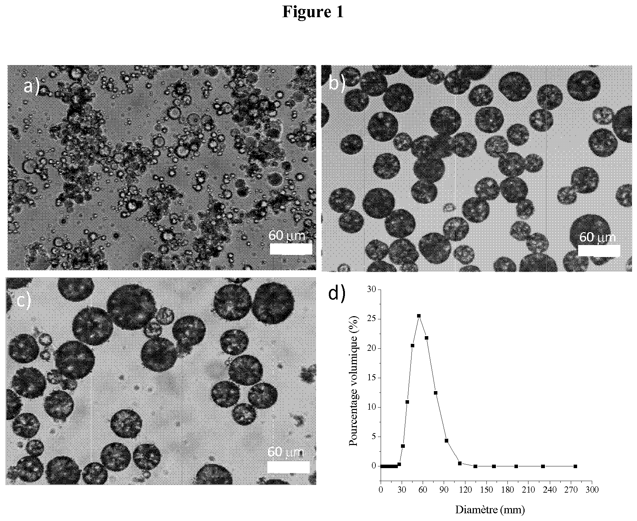

La figure 1 annexée représente une photographie en microscopie optique des différentes émulsions préparées au cours de la préparation du matériau conforme à l'invention : Figure 1a) émulsion E/H, Figure 1b) : émulsion double E/H/E à température ambiante avant l'étape de minéralisation, Figure 1c) émulsion double E/H/E à température ambiante après l'étape de minéralisation ; Figure 1d) : courbe montrant la distribution de taille des particules du matériau final (pourcentage en volume (%) en fonction du diamètre des particules (mm)).

Il a ensuite été vérifié qu'une augmentation de la température engendrait la rupture des enveloppes de silice et la libération du la phase grasse en fusion ainsi que de la phase aqueuse PA1.

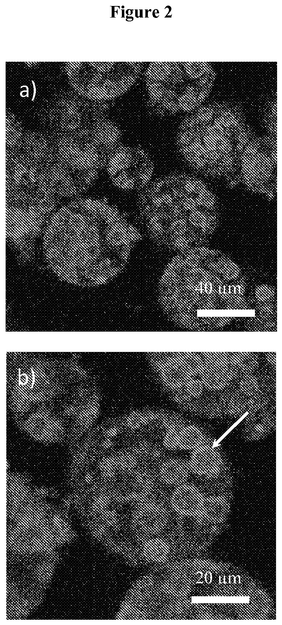

La figure 2 annexée est une image prise en microscopie confocale à épifluorescence du matériau à température ambiante, c'est-à-dire à une température à laquelle la phase grasse est à l'état solide, et à deux grossissements différents. Sur cette figure, la flèche blanche désigne l'interface eau/huile des inclusions de gouttelettes de phase aqueuse PA1 chacune encapsulée par une enveloppe de silice. On voit bien, grâce à la Rhodamine B ajoutée dans la phase aqueuse PA1, la structure du matériau conforme à l'invention. La rhodamine B est exclusivement présente dans les gouttelettes d'eau encapsulée par une enveloppe de silice, la concentration en rhodamine étant par ailleurs plus élevée au fur et à mesure que l'on se rapproche de l'interface eau/huile, lesdites gouttelettes d'eau encapsulées étant dispersées dans la phase grasse elle-même encapsulée par une enveloppe de silice.

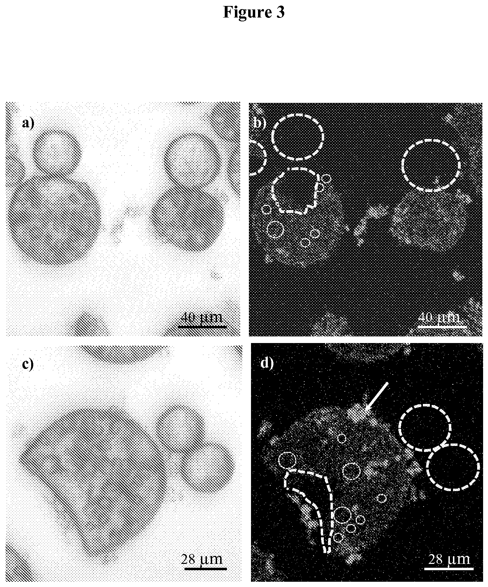

Le matériau a ensuite été porté à une température de 65°C en élevant progressivement la température à raison de 2°C par minute. Le matériau a ensuite à nouveau été observé en microscopie confocale après retour à la température ambiante. Les photos correspondantes sont données sur la figure 3 annexée sur laquelle les figures 3a et 3c sont des vues prises sans fluorescence et les figures 3b et 3d avec fluorescence, à deux grossissements différents. Sur cette figure, les traits blancs épais discontinus indiquent la présence de la phase grasse exempte de fluorescence, les traits blancs fins discontinus indiquent l'empreinte des gouttelettes d'eau celles-ci étant maintenant exemptes de fluorescence, et la flèche blanche indique la présence de quelques gouttelettes d'eau minéralisées à l'extérieur de l'enveloppe externe après sa rupture. On peut donc voir que le traitement thermique entraîne l'expulsion de la phase grasse exempte de rhodamine B par rupture de l'enveloppe de silice entourant initialement chaque globule de phase grasse. On peut également observer sur les figures 3a et 3c, quelques gouttelettes dans fluorescence correspondant en fait aux empreintes laissées par les gouttelettes d'eau qui étaient initialement encapsulées dans les enveloppes de silice internes et qui se sont répandu après rupture de ces enveloppes de silice internes. Ceci prouve que le TEOS a migré à l'intérieur des globules de phase grasse de l'émulsion double E/H/E jusqu'à l'interface huile/eau pour former une enveloppe de silice interne autour de chacune des gouttelettes de phase aqueuse PA1 dispersées dans la phase grasse et que l'expansion thermique de la phase grasse par élévation de sa température provoque à la fois la rupture de l'enveloppe externe de silice entourant les globules de phase grasse ainsi que la rupture des enveloppes de silice interne entourant les gouttelettes de phase aqueuse PA1 dispersées dans la phase grasse.

Il est ainsi possible de libérer simultanément des substances lipophiles et hydrophiles qui seraient respectivement contenues dans la phase grasse et dans la phase aqueuse PA1 par simple élévation de la température des matériaux conformes à l'invention.

**Revendications**

1. Matériau sous forme de particules solides de diamètre variant de 1 $\mu$m à 1 cm constituées d'une enveloppe continue $E_{Ext}$ comprenant au moins un oxyde de silicium, ladite enveloppe $E_{Ext}$ emprisonnant au moins une phase grasse, ledit matériau étant **caractérisé en ce que** ladite phase grasse est solide à une température de 20°C et comprend de 50 à 99,9% en masse par rapport à la masse de ladite phase grasse d'une huile cristallisable ayant une température de fusion ($T_F$) inférieure à 100°C, et **en ce que** ladite phase grasse renferme au moins une substance d'intérêt lipophile $S_L$ et au moins une inclusion comprenant une enveloppe continue $E_{Int}$ comprenant au moins un oxyde de silicium, ladite enveloppe $E_{Int}$ emprisonnant une phase aqueuse comprenant au moins une substance d'intérêt hydrophile $S_H$.

2. Matériau selon la revendication 1, **caractérisé en ce que** l'huile cristallisable est choisie parmi les matières grasses et les mélanges de matières grasses, d'origine naturelle ou synthétique, dont le point de fusion est supérieur à 15°C.

3. Matériau selon l'une quelconque des revendications précédentes, **caractérisé en ce que** l'huile cristallisable est choisie parmi les paraffines, les triglycérides ; les acides gras ; les colophanes ; les cires ; les huiles végétales hydrogénées ainsi que leurs mélanges ; les bitumes synthétiques ; et leurs mélanges.

4. Matériau selon l'une quelconque des revendications précédentes, **caractérisé en ce qu'**il se présente sous la forme d'une poudre de particules sphériques.

5. Matériau selon l'une quelconque des revendications précédentes, **caractérisé en ce que** l'enveloppe de silice $E_{Ext}$ a une épaisseur de 0,1 à 2 $\mu$m.

6. Matériau selon l'une quelconque des revendications précédentes, **caractérisé en ce que** la ou les enveloppes de silice $E_{Int}$ ont une épaisseur variant de 0,1 à 1 $\mu$m.

7. Matériau selon l'une quelconque des revendications précédentes, **caractérisé en ce que** les enveloppes $E_{Ext}$ et/ou $E_{Int}$ comprennent en outre un ou plusieurs oxydes métalliques de formule $MeO_2$ dans laquelle Me est un métal choisi parmi Zr, Ti, Th, Nb, Ta, V, W et Al.

8. Matériau selon l'une quelconque des revendications précédentes, **caractérisé en ce que** la substance d'intérêt est choisie parmi les médicaments, les principes actifs utilisables en cosmétique, les réactifs chimiques, les colorants, les pigments et les encres.

9. Matériau selon l'une quelconque des revendications précédentes, **caractérisé en ce que** la ou les substances d'intérêt $S_L$ représentent de 0,001 à 50 % en masse de la masse totale de la phase grasse.

10. Matériau selon l'une quelconque des revendications précédentes, **caractérisé en ce que** la ou les substances d'intérêt $S_H$ représentent de 0,001 à 50 % en masse de la masse totale de la phase aqueuse contenue dans la ou les inclusions présentes au sein de la phase grasse.

11. Procédé de préparation d'un matériau tel que défini à l'une quelconque des revendications 1 à 10, **caractérisé en ce qu'**il comporte les étapes suivantes consistant :

    1) dans une première étape, à porter une phase grasse comprenant de 50 à 99,9 % en masse par rapport à la masse de ladite phase grasse d'une huile cristallisable HC solide ayant une température de fusion $T_F$ inférieure à 100°C à une température $T_{HC}$ telle que $T_{HC}$ est supérieure à $T_F$, pour obtenir une huile cristallisable à l'état liquide ;

    2) dans une deuxième étape, à incorporer à la phase grasse à l'état liquide au moins une substance d'intérêt lipophile $S_L$ et des particules solides colloïdales P1 ;

    3) dans une troisième étape, à mettre en contact ladite phase grasse à l'état liquide avec une première phase aqueuse PA1 préalablement portée à une température $T_{PA1}$ telle que $T_{PA1}$ est supérieure à $T_F$, ladite phase aqueuse renfermant en outre au moins une substance d'intérêt hydrophile $S_H$ ;

    4) dans une quatrième étape, à soumettre le mélange liquide résultant de la troisième étape à une agitation mécanique pour obtenir une émulsion eau-dans-huile E/H formée de gouttelettes de phase aqueuse dispersées dans la phase grasse continue à l'état liquide et dans laquelle les particules solides colloïdales P1 sont présentes à l'interface formée entre la phase grasse continue et les gouttelettes de phase aqueuse PA1 dispersées ;

    5) dans une cinquième étape, à ajouter l'émulsion E/H obtenue ci-dessus à l'étape précédente, à une seconde phase aqueuse PA2, préalablement portée à une température $T_{PA2}$ telle que $T_{PA2}$ est supérieure à $T_F$ et renfermant en outre des particules solides colloïdales P2, ladite émulsion E/H représentant au maximum 20% en masse par rapport à la masse de la seconde phase aqueuse,

    6) dans une sixième étape, à soumettre le mélange liquide résultant de la cinquième étape à une agitation mécanique pour obtenir une émulsion double eau-dans-huile-dans-eau E/H/E formée d'une phase aqueuse continue PA2 renfermant des gouttelettes de phase grasse à l'état liquide, chacune desdites gouttelettes de phase grasse à l'état liquide renfermant au moins une gouttelette de phase aqueuse PA1 ; émulsion double dans laquelle les particules solides colloïdales P2 sont présentes à l'interface formée entre la phase aqueuse continue PA2 et les gouttelettes de phase grasse à l'état liquide dispersées ;

    7) dans une septième étape, à porter ladite émulsion double E/H/E à une température $T_{E/H/E}$ telle que $T_{E/H/E}$ est inférieure à $T_F$ pour provoquer la solidification de la phase grasse et obtenir une émulsion double E/H/E formée de globules de phase grasse à l'état solide, chacun desdits globules renfermant au moins une gouttelette de phase aqueuse PA1, lesdits globules étant dispersés dans la phase aqueuse PA2 ;

    8) dans une huitième étape, à ajouter, dans la phase aqueuse PA2 de l'émulsion double, et sous agitation mécanique, au moins un précurseur d'oxyde de silicium et une quantité suffisante d'au moins un acide pour amener les phases aqueuses PA1 et PA2 à un pH inférieur ou égal à 4 ;

9) dans une neuvième étape, à laisser l'émulsion triple E/H/E au repos, pour permettre l'hydrolyse du précurseur d'oxyde de silicium et sa condensation sous la forme d'une enveloppe d'oxyde de silicium autour desdites gouttelettes de phase aqueuse PA (minéralisation de l'émulsion) et ainsi obtenir le matériau attendu ;

10) dans une dixième étape, à séparer ledit matériau de la phase aqueuse PA2.

**12.** Procédé selon la revendication 13, **caractérisé en ce que** les particules solides colloïdales P1 et P2 sont des particules d'oxydes de métaux choisis parmi les oxydes de silicium, de titane, de zirconium et de fer ; et leur sels.

**13.** Procédé selon la revendication 12, **caractérisé en ce que** les particules solides colloïdales P1 et P2 sont de nature chimique identique et sont choisies parmi les nanoparticules d'oxyde de silicium.

**14.** Procédé selon l'une quelconque des revendications 11 à 13, **caractérisé en ce que** la quantité de particules solides colloïdales P1 varie de 0,01 % à 10% en masse par rapport à la masse totale de la phase aqueuse PA1.

**15.** Procédé selon l'une quelconque des revendications 11 à 14, **caractérisé en ce que** la quantité de particules solides colloïdales P2 varie de 0,01 % à 5% en masse par rapport à la masse totale de l'émulsion E/H.

**16.** Procédé selon l'une quelconque des revendications 11 à 15, **caractérisé en ce que** les particules solides colloïdales P1 et P2 sont fonctionnalisées par adsorption de molécules de tensioactif à leur surface.

**17.** Procédé selon l'une quelconque des revendications 11 à 16, **caractérisé en ce que** les précurseurs d'oxyde de silicium sont des alcoxydes de silicium choisis parmi le tetraméthoxyorthosilane, le tetraéthoxyorthosilane, le diméthyldiéthoxysilane, le (3-mercaptopropyl)triméthoxysilane, le (3-aminopropyl)triéthoxysilane, le N-(3-trimethoxysilylpropyl)pyrrole, le 3-(2,4-dinitrophénylamino)-propyltriéthoxysilane, le N-(2-aminoéthyl)-3-aminopropyltriméthoxysilane, le phényltriéthoxysilane, le méthyltriéthoxysilane et leurs mélanges.

**18.** Procédé selon l'une quelconque des revendications 11 à 17, **caractérisé en ce que** la quantité de précurseur d'oxyde de silicium varie de 0,005 à 4 M/m$^2$ de surface des globules de la phase dispersée de l'émulsion et des gouttelettes de phase aqueuse PA1 de l'émulsion.

**19.** Utilisation d'un matériau tel que défini à l'une quelconque des revendications 1 à 10, sous forme de poudre ou de dispersion dans un solvant, pour la délivrance thermostimulée et simultanée d'au moins une substance d'intérêt lipophile et d'au moins une substance d'intérêt hydrophile.

**20.** Utilisation selon la revendication 19, **caractérisée en ce que** la délivrance des substances d'intérêt lipophile et hydrophile est obtenue par expansion thermique de la phase grasse induisant la rupture de l'enveloppe entourant la phase grasse, ainsi que la rupture de chacune des enveloppes de silice entourant chacune des gouttelettes de phase aqueuse PA1 dispersées dans la phase grasse, sous l'effet d'une élévation de la température du matériau à une température de délivrance $T_D$ telle que $T_D > T_F$.

**21.** Utilisation d'un matériau tel que défini à l'une quelconque des revendications 1 à 10, à titre d'ingrédient, pour la préparation de produits pharmaceutiques, cosmétiques ou alimentaires.

**22.** Produits pharmaceutiques, cosmétiques ou alimentaires, **caractérisés en ce qu'**ils renferment, à titre d'ingrédient, au moins un matériau tel que défini à l'une quelconque des revendications 1 à 10.

**Patentansprüche**

**1.** Material in Form von festen Partikeln mit variierendem Durchmesser von 1 μm bis 1 cm, gebildet von einer kontinuierlichen Hülle $E_{Ext}$, umfassend mindestens ein Siliziumoxid, wobei die Hülle $E_{Ext}$ mindestens eine Fettphase einschließt, wobei das Material **dadurch gekennzeichnet ist, dass** die Fettphase bei der Lagertemperatur des Materials fest ist und 50 bis 99,9 Ma% in Bezug zu der Masse der Fettphase eines kristallisierbaren Öls mit einer Schmelztemperatur ($T_F$) unter 100 °C umfasst, und dass die Fettphase mindestens eine lipophile Substanz von Interesse $S_L$ und mindestens eine Inklusion einschließt, umfassend eine kontinuierliche Hülle $E_{Int}$, umfassend mindestens ein Siliziumoxid, wobei die Hülle $E_{Int}$ eine Wasserphase einschließt, die mindestens eine hydrophile Substanz von Interesse $S_H$ umfasst.

**2.** Material nach Anspruch 1, **dadurch gekennzeichnet, dass** das kristallisierbare Öl aus den Fetten und den Fettgemischen natürlichen oder synthetischen Ursprungs ausgewählt ist, deren Schmelzpunkt über 15 °C liegt.

3. Material nach einem der vorangehenden Ansprüche, **dadurch gekennzeichnet, dass** das kristallisierbare Öl aus den Paraffinen, den Triglyceriden, den Fettsäuren, Kolophonium, den Wachsen, den hydrogenierten pflanzlichen Ölen sowie ihren Gemischen, den synthetischen Bitumen und ihren Gemischen ausgewählt ist.

4. Material nach einem der vorangehenden Ansprüche, **dadurch gekennzeichnet, dass** es in Form eines Pulvers sphärischer Partikel vorliegt.

5. Material nach einem der vorangehenden Ansprüche, **dadurch gekennzeichnet, dass** die Siliziumoxidhülle $E_{Ext}$ eine Stärke von 0,1 bis 2 $\mu$m hat.

6. Material nach einem der vorangehenden Ansprüche, **dadurch gekennzeichnet, dass** die Siliziumoxidhülle(n) $E_{Int}$ eine Stärke hat/haben, die von 0,1 bis 1 $\mu$m schwankt.

7. Material nach einem der vorangehenden Ansprüche, **dadurch gekennzeichnet, dass** die Hüllen $E_{EXT}$ und/oder $E_{Int}$ ferner ein oder mehrere Metalloxide der Formel $MeO_2$ umfassen, in welcher Me ein Metall ist, das aus Zr, Ti, Th, Nb, Ta, V, W und Al ausgewählt ist.

8. Material nach einem der vorangehenden Ansprüche, **dadurch gekennzeichnet, dass** die Substanz von Interesse aus den Arzneimitteln, den in der Kosmetik verwendbaren Wirkstoffen, den chemischen Reagenzien, den Farbstoffen, den Pigmenten und den Farben ausgewählt ist.

9. Material nach einem der vorangehenden Ansprüche, **dadurch gekennzeichnet, dass** die Substanz(en) von Interesse $S_L$ 0,001 bis 50 Ma% der Gesamtmasse der Fettphase darstellt/darstellen.

10. Material nach einem der vorangehenden Ansprüche, **dadurch gekennzeichnet, dass** die Substanz(en) von Interesse $S_H$ 0,001 bis 50 Ma% der Gesamtmasse der Wasserphase darstellt/darstellen, die in der oder den Einschlüssen innerhalb der Fettphase enthalten ist.

11. Verfahren zur Herstellung eines Materials nach einem der Ansprüche 1 bis 10, **dadurch gekennzeichnet, dass** es die folgenden Schritte umfasst, die darin bestehen:

1) in einem ersten Schritt, Verbringen einer Fettphase, umfassend 50 bis 99,9 Ma% in Bezug zu der Masse der Fettphase eines festen kristallisierbaren Öls (HC) mit einer Schmelztemperatur $T_F$ unter 100 °C, auf eine Temperatur $T_{HC}$ derart, dass $T_{HC}$ höher als $T_F$ ist, um ein kristallisierbares Öl in flüssigem Zustand zu erhalten,

2) in einem zweiten Schritt, Inkorporieren in die Fettphase in flüssigem Zustand mindestens einer lipophilen Substanz von Interesse ($S_L$) und kolloidaler fester Partikel P1,

3) in einem dritten Schritt, Inkontaktversetzen der Fettphase in flüssigem Zustand mit einer ersten Wasserphase PA1, die zuvor auf eine Temperatur $T_{PA1}$ verbracht wurde, derart, dass $T_{PA1}$ höher als $T_F$ ist, wobei die Wasserphase ferner mindestens eine hydrophile Substanz von Interesse $S_H$ einschließt,

4) in einem vierten Schritt, mechanisches Rühren des flüssigen Gemischs aus Schritt drei, um eine Wasser-in-Öl-Emulsion W/Ö zu erhalten, die von Wasserphasentröpfchen gebildet ist, die in der kontinuierlichen Fettphase in flüssigem Zustand dispergiert sind und in welcher die kolloidalen festen Partikel P1 an der Schnittstelle, gebildet zwischen der kontinuierlichen Fettphase und den dispergierten Wasserphasentröpfchen PA1, vorhanden sind,

5) in einem fünften Schritt, Hinzufügen der in obigem Schritt erhaltenen W/Ö-Emulsion zu einer zweiten Wasserphase PA2, die zuvor auf eine Temperatur $T_{PA2}$ verbracht wurde, derart, dass $T_{PA2}$ höher als $T_F$ ist und ferner kolloidale feste Partikel P2 einschließt, wobei die W/Ö-Emulsion maximal 20 Ma% in Bezug zu der Masse der zweiten Wasserphase darstellt,

6) in einem sechsten Schritt, mechanisches Rühren des flüssigen Gemischs aus Schritt fünf, um eine Wasser-in-Öl-in-Wasser-Doppelemulsion W/Ö/W zu erhalten, gebildet von einer kontinuierlichen Wasserphase PA2, die Fettphasentröpfchen in flüssigem Zustand einschließt, wobei jedes der Fettphasentröpfchen in flüssigem Zustand mindestens ein Wasserphasentröpfchen PA1 einschließt, wobei die Doppelemulsion, in der die kolloidalen festen Partikel P2 an der Schnittstelle, gebildet zwischen der kontinuierlichen Wasserphase PA2 und den dispergierten Fettphasentröpfchen in flüssigem Zustand, vorhanden sind,

7) in einem siebenten Schritt, Verbringen der Doppelemulsion W/Ö/W auf eine Temperatur $T_{E/H/E}$ derart, dass $T_{E/H/E}$ unter $T_F$ ist, um die Verfestigung der Fettphase zu bewirken und eine Doppelemulsion W/Ö/W zu erhalten, die von Fettphasenkörperchen in festem Zustand gebildet ist, wobei jedes der Körperchen mindestens ein Wasserphasentröpfchen PA1 einschließt, wobei die Körperchen in der Wasserphase PA2 dispergiert sind,

8) in einem achten Schritt, Hinzufügen, zu der Wasserphase PA2 der Doppelemulsion und unter mechanischem Rühren, mindestens eines Siliziumoxidvorläufers und einer ausreichenden Menge mindestens einer Säure, um die Wasserphasen PA1 und PA2 auf einen pH von unter oder gleich 4 zu führen,

9) in einem neunten Schritt, Ruhenlassen der Dreifachemulsion W/Ö/W, um die Hydrolyse des Siliziumoxidvorläufers und seine Kondensation in Form einer Siliziumoxidhülle um die Wasserphasentröpfchen PA (Mineralisierung der Emulsion) zu erlauben und damit das erwartete Material zu erhalten,

10) in einem zehnten Schritt, Separieren des Materials der Wasserphase PA2.

**12.** Verfahren nach Anspruch 13, **dadurch gekennzeichnet, dass** die kolloidalen festen Partikel P1 und P2 Metalloxidpartikel sind, die aus den Oxiden von Silizium, Titan, Zirkonium und Eisen und ihren Salzen ausgewählt sind.

**13.** Verfahren nach Anspruch 12, **dadurch gekennzeichnet, dass** die kolloidalen festen Partikel P1 und P2 chemisch identischer Art sind und aus den Siliziumoxid-Nanopartikeln ausgewählt sind.

**14.** Verfahren nach einem der Ansprüche 11 bis 13, **dadurch gekennzeichnet, dass** die Menge kolloidaler fester Partikel P1 von 0,01 bis 10 Ma% in Bezug zu der Gesamtmasse der Wasserphase PA1 schwankt.

**15.** Verfahren nach einem der Ansprüche 11 bis 14, **dadurch gekennzeichnet, dass** die Menge kolloidaler fester Partikel P2 von 0,01 bis 5 Ma% in Bezug zu der Gesamtmasse der W/Ö-Emulsion schwankt.

**16.** Verfahren nach einem der Ansprüche 11 bis 15, **dadurch gekennzeichnet, dass** die kolloidalen festen Partikel P1 und P2 durch Adsorption von Tensidmolekülen auf ihrer Oberfläche funktionalisiert sind.

**17.** Verfahren nach einem der Ansprüche 11 bis 16, **dadurch gekennzeichnet, dass** die Siliziumoxidvorläufer Siliziumalcoxide sind, ausgewählt aus dem Tetramethoxyorthosilan, dem Tetraethoxyorthosilan, dem Dimethyldiethoxysilan, dem (3-Mercaptopropyl)trimethoxysilan, dem (3-Aminopropyl)triethoxysilan, dem N-(3-trimethoxysilylpropyl)pyrrol, dem 3-(2,4-Dinitrophenylamino)-propyltriethoxysilan, dem N-(2-aminoethyl)-3-aminopropyltrimethoxysilan, dem Phenyltriethoxysilan, dem Methyltriethoxysilan und ihren Gemischen.

**18.** Verfahren nach einem der Ansprüche 11 bis 17, **dadurch gekennzeichnet, dass** die Menge Siliziumoxidvorläufer von 0,005 bis 4 M/m² Oberfläche der Körperchen der dispergierten Phase der Emulsion und der Wasserphasentröpfchen PA1 der Emulsion schwankt.

**19.** Verwendung eines Materials nach einem der Ansprüche 1 bis 10 in Form von Pulver oder Dispersion in einem Lösungsmittel für die thermisch stimulierte und gleichzeitige Abgabe mindestens einer lipophilen Substanz von Interesse und mindestens einer hydrophilen Substanz von Interesse.

**20.** Verwendung nach Anspruch 19, **dadurch gekennzeichnet, dass** die Abgabe der lipophilen und hydrophilen Substanzen von Interesse durch thermische Expansion der Fettphase erfolgt, die den Riss der Hülle, die die Fettphase umgibt, induziert, sowie den Riss jeder der Siliziumoxidhüllen, die jedes der in der Fettphase dispergierten Wasserphasentröpfchen PA1 umgeben, infolge der Temperaturerhöhung des Materials auf eine Abgabetemperatur $T_D$, die derart ist, dass $T_D > T_F$.

**21.** Verwendung eines Materials nach einem der Ansprüche 1 bis 10 als Zutat für die Zubereitung von pharmazeutischen, kosmetischen oder Lebensmittelprodukten.

**22.** Pharmazeutische, kosmetische oder Lebensmittelprodukte, **dadurch gekennzeichnet, dass** sie als Zutat mindestens ein Material nach einem der Ansprüche 1 bis 10 enthalten.

**Claims**

**1.** Material in the form of solid particles having a diameter varying from 1 µm to 1 cm, composed of a continuous shell $E_{ext}$ comprising at least one silicon oxide, said shell $E_{ext}$ trapping at least one fat phase, said material being **characterized in that** said fat phase is solid at a temperature of 20 °C and comprises from 50 to 99.9 % by weight, relative to the weight of said fat phase, of a crystallisable oil having a melting temperature ($T_M$) lower than 100°C, and **in that** said fat phase contains at least one lipophilic substance of interest $S_L$ and at least one inclusion comprising a continuous shell $E_{int}$ comprising at least one silicon oxide, said shell $E_{int}$ trapping an aqueous phase comprising at least one hydrophilic substance of interest $S_H$.

**2.** The material according to claim 1, **characterized in that** the crystallisable oil is selected from among fats and mixtures of fats, of natural or synthetic origin, having a melting point higher than 15°C.

**3.** The material according to any of the preceding claims, **characterized in that** the crystallisable oil is selected from among paraffins, triglycerides; fatty acids; rosins; waxes; hydrogenated vegetable oils and mixtures thereof; synthetic bitumen; and mixtures thereof.

4. The material according to any of the preceding claims, **characterized in that** it is in the form of a powder of spherical particles.

5. The material according to any of the preceding claims, **characterized in that** the silica shell $E_{ext}$ has a thickness of 0.1 to 2 $\mu$m.

6. The material according to any of the preceding claims, **characterized in that** the silica shell(s) $E_{int}$ have a thickness varying from 0.1 to 1 $\mu$m.

7. The material according to any of the preceding claims, **characterized in that** the shells $E_{ext}$ and/or $E_{int}$ also comprise one or more metal oxides of formula $MeO_2$ where Me is a metal selected from among Zr, Ti, Th, Nb, Ta, V, W and Al.

8. The material according to any of the preceding claims, **characterized in that** the substance of interest is selected from among medicinal products, active ingredients for use in cosmetics, chemical reagents, dyes, pigments and inks.

9. The material according to any of the preceding claims, **characterized in that** the substance(s) of interest $S_L$ represent from 0.001 to 50 % by weight of the total weight of the fat phase.

10. The material according to any of the preceding claims, **characterized in that** the substance(s) of interest $S_H$ represent from 0.001 to 50 % by weight of the total weight of the aqueous phase contained in the inclusion(s) included in the fat phase.

11. Method for preparing a material such as defined in any of claims 1 to 10, **characterized in that** it comprises the following steps of:

1) at a first step, bringing the fat phase - comprising from 50 to 99.9 % by weight, relative to the weight of said fat phase, of a solid crystallisable oil HC having a melting temperature $T_M$ lower than 100°C - to a temperature $T_{HC}$ such that $T_{HC}$ is higher than $T_M$ to obtain a crystallisable oil in the liquid state;

2) at a second step, incorporating in the fat phase in the liquid state at least one lipophilic substance of interest $S_L$ and solid colloidal particles P1;

3) at a third step, placing said fat phase in the liquid state in contact with a first aqueous phase PA1 previously brought to a temperature $T_{PA1}$ such that $T_{PA1}$ is higher than $T_M$, said aqueous phase also containing at least one hydrophilic substance of interest $S_H$;

4) at a fourth step, subjecting the liquid mixture resulting from the third step to mechanical agitation to obtain a water-in-oil emulsion W/O formed of droplets of aqueous phase dispersed in the continuous fat phase in the liquid state, and wherein the solid colloidal particles P1 are present at the interface formed between the continuous fat phase and the dispersed droplets of aqueous phase PA1;

5) at a fifth step, adding the W/O emulsion obtained above at the preceding step to a second aqueous phase PA2 previously brought to a temperature $T_{PA2}$ such that $T_{PA2}$ is higher than $T_M$ and also containing solid colloidal particles P2, said W/O emulsion representing at most 20 % by weight relative to the weight of the second aqueous phase;

6) at a sixth step, subjecting the liquid mixture resulting from the fifth step to mechanical agitation to obtain a dual water-in-oil-in-water emulsion W/O/W formed of a continuous aqueous phase PA2 containing droplets of fat phase in the liquid state, each of said droplets of fat phase in the liquid state containing at least one droplet of aqueous phase PA1; a dual emulsion in which the solid colloidal particles P2 are present at the interface formed between the continuous aqueous phase PA2 and the dispersed droplets of fat phase in the liquid state;

7) at a seventh step, bringing said dual emulsion W/O/W to a temperature $T_{W/O/W}$ such that $T_{W/O/W}$ is lower than $T_M$ to cause solidification of the fat phase and obtain a dual emulsion W/O/W formed of globules of fat phase in the solid state, each of said globules containing at least one droplet of aqueous phase PA1, said globules being dispersed in the aqueous phase PA2;

8) at an eighth step, adding to the aqueous phase PA2 of the dual emulsion and under mechanical agitation at least one precursor of silicon oxide and a sufficient amount of at least one acid to bring the aqueous phases PA1 and PA2 to a pH of 4 or lower;

9) at a ninth step, leaving the triple emulsion W/O/W to stand, to allow hydrolysis of the silicon oxide precursor and condensing thereof into the form of a shell of silicon oxide around said droplets of aqueous phase PA (mineralisation of the emulsion) and thereby obtain the expected material;

10) at a tenth step, separating said material from the aqueous phase PA2.

12. The method according to claim 13, **characterized in that** the solid colloidal particles P1 and P2 are particles of metal oxides selected from among the oxides of silicon, titanium, zirconium and iron; and the salts thereof.

13. The method according to claim 12, **characterized in that** the solid colloidal particles P1 and P2 are of same chemical type and are selected from among nanoparticles of silicon oxide.

14. The method according to any of claims 11 to 13, **characterized in that** the quantity of solid colloidal particles P1 varies from 0.01 % to 10 % by weight relative to the total weight of the aqueous phase PA1.

15. The method according to any of claims 11 to 14, **characterized in that** the quantity of solid colloidal particles P2 varies from 0.01 % to 5 % by weight relative to the total weight of the emulsion W/0.

16. The method according to any of claims 11 to 15, **characterized in that** the solid colloidal particles P1 and P2 are functionalized by adsorption of surfactant molecules on the surface thereof.

17. The method according to any of claims 11 to 16, **characterized in that** the silicon oxide precursors are silicon alkoxides selected from among tetramethoxyorthosilane, tetraethoxyorthosilane, dimethlydiethoxysilane, (3-mercaptopropyl)trimethoxysilane, (3-aminopropyl)triethoxysilane, N-(3-trimethoxysilylpropyl)pyrrole, 3-(2,4-dinitrophenylamino)-propyltriethoxysilane, N-(2-aminoethyl)-3-aminopropyltrimethoxysilane, phenyltriethoxysilane, methyltriethoxysilane and mixtures thereof.

18. The method according to any of claims 11 to 17, **characterized in that** the amount of silicon oxide precursor varies from 0.005 to 4 $M/m^2$ surface areas of the globules of dispersed phase of the emulsion and of the droplets of aqueous phase PA1 of the emulsion.

19. Use of a material such as defined in any of claims 1 to 10, in the form of a powder or dispersion in a solvent, for the simultaneous thermostimulated delivery of at least one lipophilic substance of interest and at least one hydrophilic substance of interest.

20. The use according to claim 19, **characterized in that** the delivery of the lipophilic and hydrophilic substances of interest is obtained by thermal expansion of the fat phase causing rupture of the shell surrounding the fat phase, and rupture of each of the silica shells surrounding each of the droplets of aqueous phase PA1 dispersed in the fat phase, under the effect of a rise in temperature of the material to a delivery temperature $T_D$ such that $T_D > T_M$.

21. Use of a material such as defined in any of claims 1 to 10, as ingredient for the preparation of pharmaceutical, cosmetic or food products.

22. Pharmaceutical, cosmetic or food products, **characterized in that** as ingredient they contain at least one material such as defined in any of claims 1 to 10.

**Figure 1**

**Figure 2**

Figure 3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2948581 A

**Littérature non-brevet citée dans la description**

- **MATHIEU DESTRIBATS.** Thermostimulable Wax@SiO2 Core-Shell Particles. *Langmuir,* 02 Février 2010, vol. 26 (3), 1734-1742